# EUROPÄISCHE PATENTSCHRIFT

(11) **EP 3 559 621 B1**
(45) Veröffentlichungstag und Bekanntmachung des Hinweises auf die Patenterteilung: **20.11.2024**
(21) Anmeldenummer: 17811968.1
(22) Anmeldetag: 15.12.2017
(51) Int. Cl.: A61B 5/01, A61B 5/00, G04G 21/02, G06F 1/16, G01K 1/16, G01K 13/20, G04G 17/00, G04G 17/04

(54) **SENSOREINHEIT FÜR EIN TRAGBARES COMPUTERSYSTEM UND INTEGRATION DER SENSOREINHEIT**
SENSOR UNIT FOR A PORTABLE COMPUTER SYSTEM AND INTEGRATION OF THE SENSOR UNIT
UNITÉ CAPTEUR POUR UN SYSTÈME INFORMATIQUE PORTABLE ET INTÉGRATION DE L'UNITÉ CAPTEUR

(30) Priorität: 21.12.2016 CH 16952016
(43) Veröffentlichungstag der Anmeldung: 30.10.2019
(73) Patentinhaber: Greenteg AG, 8153 Rümlang (CH)
(72) Erfinder: ZAHNER, Michele, 8037 Zürich (CH); HELBLING, Thomas, 8500 Frauenfeld (CH); DURRER, Lukas, 9642 Ebnat-Kappel (CH); SCHWYTER, Etienne, 8055 Zürich (CH)
(74) Vertreter: Prins Intellectual Property AG
(86) Internationale Anmeldenummer: PCT/EP2017/082994
(87) Internationale Veröffentlichungsnummer: WO 2018/114653

(56) Entgegenhaltungen:
- WO-A2-02/078538
- DE-A1- 102015 206 938
- US-A1- 2004 039 254
- US-A1- 2007 100 666
- ANONYMOUS: "RdF HFS-A // Heat Flux Sensors", 21 April 2003 (2003-04-21), XP093095238, Retrieved from the Internet <URL:https://web.archive.org/web/20030421111452/https://www.rdfcorp.com/products/hflux/hfs-a_02.shtml> [retrieved on 20231026]
- ANONYMOUS: "Simplified Heat Flow Measurement: Construction & Principle of Operation", 1 April 2003 (2003-04-01), XP055448075, Retrieved from the Internet <URL:https://web.archive.org/web/20030401070308/http://www.rdfcorp.com/anotes/pa-hfs/pa-hfs_02.shtml> [retrieved on 20180206]

## Beschreibung

### Technisches Gebiet

Die vorliegende Erfindung beschreibt eine Sensoreinheit zur Bestimmung der Körperkerntemperatur durch ausserhalb des Körpers auf einer Oberfläche ermittelbare Messwerte, umfassend mindestens einen monolithischen Wärmeflusssensor und mindestens einen Temperatursensor, ein Integrationsverfahren einer Sensoreinheit und die Herstellung einer Sensoreinheit, umfassend mindestens einen monolithischen Wärmeflusssensor, mindestens einen Temperatursensor und einen Analog/Digital Wandler, auf einer Leiterplatte und Integration der Sensoreinheit in ein Gehäuse eines tragbaren Computersystems oder in ein Klebepad zum Anbringen auf der Haut.

### Stand der Technik

Tragbare technische Datenverarbeitungsvorrichtungen mit Messmöglichkeiten, sind in verschiedenen Ausgestaltungen mit oder ohne Gehäuse als Teil von Kleidung oder in Form von Schmuck bekannt. Mit diesen tragbaren Computersystemen oder wearable Computern beschäftigt sich das Forschungsgebiet "Wearable Computing". Die wearable Computer bzw. tragbaren Computersysteme weisen wenigstens eine Sensoreinheit auf, mit welcher Daten über mindestens einen Sensor aus der realen Welt erfasst werden. Die Messdaten werden dann entweder im tragbaren Computersystem selbst verarbeitet oder an einen anderen Rechner, insbesondere an ein Smartphone übertragen. Der Benutzer kann die aufgezeichneten Daten einsehen und weiterverarbeiten. Das tragbare Computersystem umfasst ein Gehäuse, in welchem eine Sensoreinheit und eine Elektronik integriert sind. Die Elektronik weist in der Regel mindestens einen Mikrokontroller und eine Auslese-/Speichereinheit auf, welche mit der Sensoreinheit verbunden sind.

Ein Beispiel für einen wearable Computer bzw. ein tragbares Computersystem ist ein Activity Tracker, auch Fitness- bzw. Gesundheits-Armband, Smart Band oder Fitness Tracker genannt. Mittels einer, in der Elektronik betriebenen Software werden Daten von mindestens einem Sensor der Sensoreinheit erfasst, weiterverarbeitet, gegebenenfalls in einem Speicher gespeichert und/oder an ein Smartphone weitergeleitet. Entsprechend können beispielsweise fitness- und gesundheitsrelevante Daten wie etwa Laufstrecken, Energieumsatz und in manchen Fällen auch Herzschlagfrequenz, Körpertemperatur, Sauerstoffgehalt im Blut oder die Schlafqualität bestimmt werden.

Hier ist die Bestimmung der Körperinnentemperatur bzw. Körperkerntemperatur mittels tragbaren Computersystems von Interesse. Auf der Haut gemessene, nicht-invasive und kontinuierliche Körperinnentemperaturbestimmung ist wichtig für die Patientenüberwachung in Spitälern, für die Vorbeugung von Hitzschlag bei Sportlern oder für die Bestimmung und Überwachung des zirkadianen Zyklus zur Diagnose von Schlaf- und andere Krankheiten.

Die Sensoreinheit des tragbaren Computersystems basiert im einfachsten Fall auf einem Temperatursensor zur Bestimmung der Hauttemperatur in Kombination mit einer thermischen Isolierung über dem Sensor und dessen Umgebung. Die Isolierung soll bezwecken, dass die Hauttemperatur möglichst nahe an die Kernkörpertemperatur geführt wird. Die Methode ist in vielen Fällen ungenau und für die Anwendung unter wechselnden äusseren Bedingungen nicht geeignet.

Zur Verbesserung der Messung der Körperkerntemperatur kann die Sensoreinheit des tragbaren Computersystems einen sogenannten Doppeltemperatursensor gemäss DE10038247 umfassen. Hierbei werden zwei Temperatursensoren durch eine thermische Isolierung mit bekanntem thermischem Widerstand voneinander getrennt. In den meisten Fällen braucht es bei dieser Methode eine Kalibrationsmessung um den thermische Widerstand zu bestimmen, welcher von der Person und auch dem Ort der Messung abhängig ist.

Die Doppelsensor-Methode hat mehrere Nachteile. Ein grosses Problem stellen die thermischen parasitären Wärmeverluste zwischen den beiden Temperatursensoren dar, welche zu starken Abweichungen führen können. Diese müssen durch thermische Isolierung und durch komplexe Kompensationsalgorithmen wie in der DE102005004933 beschrieben, behoben werden. Ein weiterer Nachteil liegt in der Grösse des Systems. Um eine gute Sensitivität erreichen zu können, muss der Temperaturunterschied in der Anwendung genügend gross sein, was bedingt, dass das Material zwischen den beiden Temperatursensoren eine gewisse minimale Dicke aufweisen muss. Dies macht das Sensorsystem eher klotzig und sperrig und weniger geeignet für die einfache Integration in Gehäuse von dem Stand der Technik.

Die bisher bekannten Sensoreinheiten zur Bestimmung der Körperkerntemperatur eignen sich bislang nicht zur einfachen Integration in das Gehäuse eines tragbaren Computersystems. Um parasitäre Wärmeverluste zu vermeiden, sind thermische Isolationen und zusätzliche Konstruktionen nötig, wodurch ein komplexer Aufbau resultiert und die Integration der Sensoreinheit in das Gehäuse eines tragbaren Computersystems erschwert wird. Ein weiterer Nachteil ist die Messungenauigkeit bei Bestimmung der Körperkerntemperatur bekannter Sensoreinheiten.

In der WO02078538 wird die Bestimmung der Körperkerntemperatur mit Hilfe eines Wärmeflusssensors vorgestellt, welcher auf der Haut eines Benutzers befestigt wird. Der Wärmeflusssensor bildet einen Teil einer Sensoreinheit, welche auch hier aufwändig an einem Computersystem befestigt werden muss. In der US 2004/0039254 A1 werden zwei Möglichkeiten eines tragbaren Computersystems mit mindestens einem Temperatur- und einem Wärmeflusssensor beschrieben. In einem Fall wird ein Wärmeflusssensor dadurch geschaffen, dass zwei Temperatursensoren auf zwei gegenüberliegenden Seiten der Leiterplatte angebracht werden, auf der auch ein Temperatursensor angebracht ist, und in dem anderen Fall wird ein monolithischer Wärmeflusssensor auf einer "erhöhten Plattform", also nicht auf der Leiterplatte angebracht, auf der der Temperatursensor montiert ist. Monolithische Wärmeflusssensoren werden von der Firma "RdF Corporation of Hudson, N.H" vertrieben, ein solcher ist z.B. in https://web.archive.org/web/20030421111452/ https://www.rdfcorp.com/products/hflux/hfs-a_02.shtml (XP093095238) offenbart.

In der WO2005092177 wird eine weitere Sensoreinheit, geeignet zur Messung der Körperkerntemperatur beschrieben, welche unter anderem einen Wärmeflusssensor umfasst und ebenfalls an ein tragbares Computersystem anschliessbar ist. Damit ist man dem Ziel, die Körperkerntemperatur möglichst exakt zu bestimmen näher gekommen, aber die Herstellung der Sensoreinheit und die Integration der Sensoreinheit im tragbaren Computersystem sind weiterhin kompliziert und umständlich.

### Darstellung der Erfindung

Die vorliegende Erfindung hat sich zur Aufgabe gestellt eine kompakte einfach installierbare Sensoreinheit zum Einbau in ein tragbares Computersystem bzw. zur Befestigung an oder in einem Klebepad zu schaffen, welche eine optimierte Bestimmung der Körperkerntemperatur erlaubt.

Es ist eine Möglichkeit geschaffen, die Herstellung der Sensoreinheit zu vereinfachen und die Befestigung der Sensoreinheit an bzw. in einem Gehäuse eines tragbaren Computersystems bzw. an oder in einem Klebepad zu vereinfachen.

Mit der vorgestellten Sensoreinheit können thermophysiologische Daten an einem Körper, insbesondere die Körperinnentemperatur bzw. Körperkerntemperatur gemessen werden, wobei Wärmeflüsse und Oberflächentemperaturen bestimmt werden. Weitere Größen können sein: Wärmeabgabe der Haut, Kalorienverbrauch, Kalorieneinahme, Blutdruck, Blutzucker, Innentemperatur an einer bestimmten Körperstelle, kompensierte Hauttemperatur etc.

Es wird insbesondere ein Verfahren beschrieben, welches die Ankopplung einer Sensoreinheit an ein Gehäuse eines tragbaren Computersystems oder die Befestigung an oder in einem Klebepad oder mittels eines Bandes mit wenigen Arbeitsschritten erlaubt.

### Kurze Beschreibung der Zeichnungen

Weitere Merkmale, Einzelheiten und Vorzüge der Erfindung ergeben sich aus der nachfolgenden Beschreibung bevorzugter Ausführungsformen der Erfindung sowie der Zeichnungen. Es sind dargestellt in
- Figuren 1a bis 1c: zeigen schematisch Schnittansichten von tragbaren Computersystemen mit integrierten Sensoreinheiten.
- Figuren 2a bis 2e: zeigen schematische Schnittansichten verschiedener Sensoreinheiten.
- Figur2f: zeigt eine schematische Schnittansicht einer Sensoreinheit, wobei die elektronischen Bauteile in einem integrierten Schaltkreis (IC) verkapselt in einem Chipgehäuse zusammengefasst sind.
- Figur 3: zeigt eine Aufsicht auf eine Sensoreinheit vor der Befestigung einer Sensoreinheitshülle.
- Figur 4a: zeigt eine Aufsicht auf die Sensoreinheit gemäss Figur 3 nach Befestigung der Sensoreinheitshülle, während
- Figur 4b: eine schematische Schnittansicht der Sensoreinheit gemäss Figur 4a entlang der Schnittlinie B-B zeigt.
- Figur 5: zeigt eine schematische Schnittansicht einer Sensoreinheit mit einer Sensoreinheitshülle in einer Aussparung in einem Klebepad.
- Figur 6a: zeigt eine schematische Schnittansicht der Fixierung einer Sensoreinheit an einem Gehäuse, während
- Figur 6b: eine schematische Schnittansicht der Sensoreinheit nach Auffüllen eines Durchgangsloches und
- Figur 6c: nach Befestigung eines Metallstabs an der Sensoreinheit in einer schematischen Schnittansicht zeigt.
- Figur 7a: zeigt eine schematische Schnittansicht eines ersten Schrittes eines Herstellungsverfahrens einer Sensoreinheit, während
- Figur 7b: eine schematische Schnittansicht einer in eine Sensoreinheitshülle eingegossenen Sensoreinheit und
- Figur 7c: eine schematische Schnittansicht der eingegossenen Sensoreinheit gemäss Figur 6b nach Einpressen in eine Aussparung in einem Gehäuse eines tragbaren Computersystems und in Kontaktbringung mit der Haut eines Benutzers zeigt.

### Beschreibung

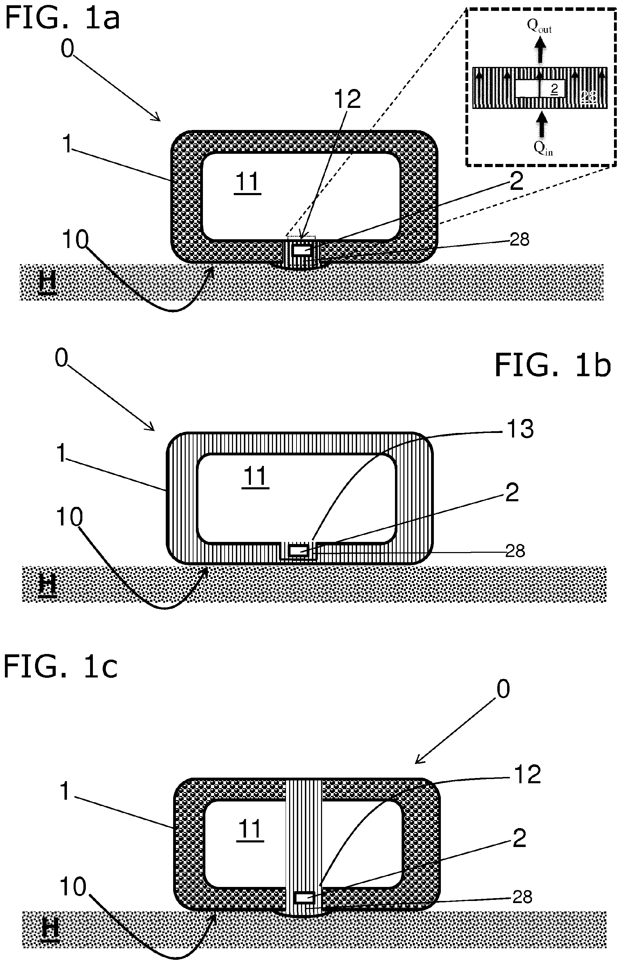

Ein tragbares Computersystem 0 ist hier in Form eines hohlen Innenraums 11 aufweisendes Armband mit einem Gehäuse 1 dargestellt. Das Gehäuse 1 wird mit einer Hautkontaktseite 10 auf die Haut H eines Benutzers aufgelegt. Vom Körper des Benutzers, über die Haut H das Gehäuse 1 des tragbaren Computersystems 0 querend bis in den Innenraum 11 tritt ein Wärmestrom auf, welcher im Einsatz von Figur 1a markiert von Qin bis Qout durch das Gehäuse 1 fliesst. Als Benutzer kommen Menschen und Tiere in Frage.

Eine Sensoreinheit 2, die im Folgenden näher spezifiziert wird, ist in das Gehäuse 1 eingelassen lösbar oder unlösbar befestigt und elektrisch mit einer nicht dargestellten Elektronik des tragbaren Computersystems 0 verbunden. Die Sensoreinheit 2 ist von einer Sensoreinheitshülle 28 mindestens teilweise umgeben in das Gehäuse 1 eingepresst bzw. eingeklebt oder eingegossen gelagert. Die Sensoreinheitshülle 28 ist aus einem thermisch leitfähigen Material, insbesondere aus Silikon hergestellt. Durch die Verwendung eines Materials mit einer ähnlichen thermischen Leitfähigkeit wie der später beschriebene Wärmeflusssensor selber, kann man den Wärmefluss homogenisieren und dadurch parasitäre Wärmeströme minimieren. Desweiteren hat eine gute thermische Leitfähigkeit zur Folge, dass die Temperatur gemessen näher der Hauttemperatur ist. Die Leitfähigkeit der Sensoreinheitshülle 28 in unserem Falle sollte zwischen 0.3 und 10 W/mK liegen. Optimal zwischen 0.8 und 3 W/mK. Aus messtechnischen Gründen, sollte siche die Sensoreinheit 2 möglichst nahe der Haut des Benutzers befinden, kann aber im Grunde überall im Wärmepfad angeordnet sein.

In der Ausführungsform gemäss Figur 1a ist ein Durchgangsloch 12, das Gehäuse 1 vollständig querend vorgesehen, in welches eine vollständig eingegossene Sensoreinheit 2 eingelassen ist. Die Sensoreinheitshülle 28 ragt etwas aus dem Durchgangsloch 12 und der Hautkontaktseite 10 heraus und kommt bei der Benutzung mit der Haut H des Benutzers in Kontakt. Der Wärmefluss von der Haut H und damit vom Körper über die Haut durch die Sensoreinheitshülle 28 und die Sensoreinheit 2 stellt sich bei der Benutzung ein und kann zur Bestimmung der Körperkerntemperatur genutzt werden.

Wie in Figur 1b gezeigt, kann auch eine Aussparung in Form eines Sackloches 13 im Gehäuse 1 des tragbaren Computersystems angeordnet sein, in welches die Sensoreinheit 2 mit teilweise umgebender Sensoreinheitshülle 28 eingebracht ist. Auch hier ist die Sensoreinheit 2 samt Sensoreinheitshülle 28 in Form eines Pfropfens in das Sackloch 13 eingesteckt und damit lösbar mit dem Gehäuse 1 verbunden.

Um die Stabilität der Lagerung der Sensoreinheit 2 mit Sensoreinheitshülle 28 im Gehäuse 1 zu steigern, kann die Sensoreinheitshülle 28 quer durch das Gehäuse 1 ragen, wie in Figur 1c gezeigt. Hiermit wird ein thermischer Pfad geschaffen, damit sich das thermische Gleichgewicht zwischen der Umgebung und dem Körper möglichst rasch einstellt. Entsprechend reicht die Sensoreinheitshülle 28 von der Hautkontaktseite 10 bis zu einer von der Hautkontaktseite 10 abgewandten Seite des Gehäuses 1 und quert das Gehäuse 1 dabei.

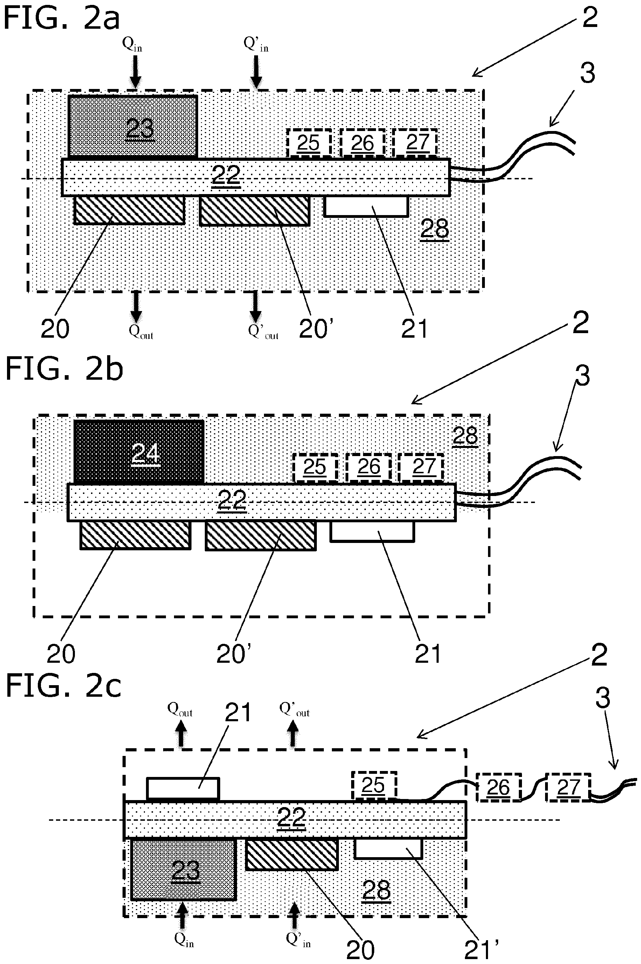

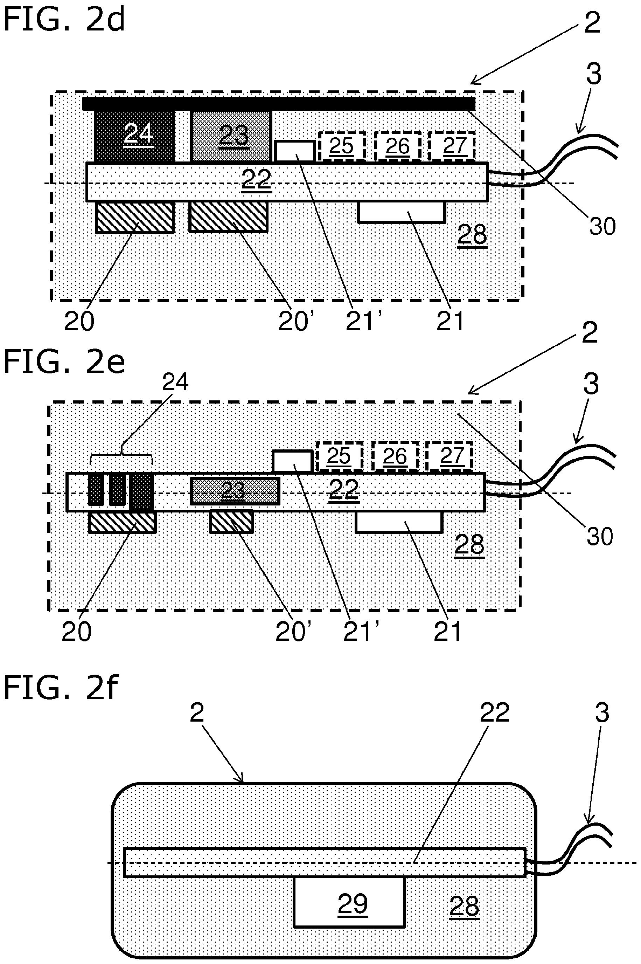

In den Figuren 2a bis 2d werden Ausführungsformen der Sensoreinheiten 2 zur Bestimmung der Körperkerntemperatur gezeigt. Ziel ist es eine Sensoreinheit 2 zu schaffen, welche möglichst kompakt, mechanisch robust ist und im besten Fall flexibel ausgestaltet ist.

Basis der Sensoreinheit 2 ist eine Leiterplatte 22 auf welcher mindestens ein Wärmeflusssensor 20 und mindestens ein Temperatursensor 21 aufgelötet sind. Die Leiterplatte 22 kann starr oder flexibel ausgeführt sein und umfasst neben Leiterbahnen aus einem elektrischen Leiter, isolierendes Material, bevorzugt faserverstärkten Kunststoff. Optimal hat das Material der mindestens einen Leiterplatte 22 eine thermische Leitfähigkeit zwischen 0.3 und 10 W/mK noch besser zwischen 0.8 und 3 W/mK, damit ein homogener Wärmefluss gewährleistet ist.

Die unterschiedlichen Sensoren 20, 21 werden auf Lötflächen auf der Leiterplatte 22 platziert und mittels Lot befestigt. Dabei sind der mindestens eine Wärmeflusssensor 20 und der mindestens eine Temperatursensor 21 entlang einer Längsrichtung voneinander örtlich beabstandet angeordnet. Wärmeflusssensor 20 und Temperatursensor 21 sind auf derselben Seite der Leiterplatte 22 befestigt.

Auch weitere Bauteile, insbesondere Blöcke aus thermisch isolierenden Materialien 23 und thermischen Kapazitäten 24, werden auf der Leitplatte 22 befestigt. Die thermische Isolation 23 und die thermische Kapazität 24 werden mit einem Kleber, beispielsweise Acryl, Epoxy oder Polyurethan auf der Leiterplatte 22 festgeklebt oder wenn es das Material zulässt, auch aufgelötet

Um die thermischen Übergangswiderstände zwischen Bauteilen möglichst gering zu halten, beispielsweise zwischen Wärmeflusssensor 20 und thermischer Kapazität 24, sollte im Falle einer Verklebung ein thermisch leitfähiger Kleber verwendet werden.

Ebenso elektronische Bauteile, wie ein Analog/Digital Wandler 25, ein Mikrokontroller 26 und eine Auslese-/Speichereinheit 27 können auf der Leiterplatte 22 elektrisch leitend verbunden befestigt werden. Zur Erreichung ausreichend hoher Messsignale ist es allerdings ausreichend nur den Analog/Digital Wandler 25 auf der Leiterplatte 22 zu befestigen und Mikrokontroller 26 und eine Auslese-/Speichereinheit 27 durch Kabel oder sogenannte starrflex-Leiterplatten, eine Schichtanordnung von Kunststoff und Kupferlagen, welche eine flexible Verbundleiterplatte bilden, beabstandet von der Leiterplatte 22 anzuordnen.

Von der Leiterplatte 22 wegführend sind Verbindungskabel 3 angeordnet, welche die Messsignale bzw. verarbeitete Signale an eine Elektronik des tragbaren Computersystems 0 weiterleiten. Wahlweise können die Verbindungskabel 3 auch von flexiblen Leiterplatten oder Leiterbahnen gebildet werden, womit die elektronischen Bauteile verbindbar sind.

Um die Stabilität der Sensoreinheit 2 zu erhöhen und einen homogenen Wärmefluss zu erreichen, ist die mit Bauteilen bestückte Leiterplatte 22 mindestens teilweise von der Sensoreinheitshülle 28 umgeben. Während die Varianten gemäss Figur 2a und 2d eine vollständig in eine Sensoreinheitshülle 28 eingegossene Sensoreinheit 2 zeigen, sind die anderen Ausführungsformen hier nur in ihrem späteren zur Haut H zugewandten Seite der Sensoreinheit 2 mit einer Sensoreinheitshülle 28 versehen.

Die Sensoreinheitshülle 28 muss nicht zwingend nur aus einem Material bestehen. Zum Beispiel kann auf der Haut anliegend ein hautverträglicheres Silikon angeordnet sein und anschliessend kommt thermisch leitender Silikon bis zur Leiterplatte 22. Die Leiterplatte 22 selber hat eine ähnliche thermische Leitfähigkeit wie die Sensroeinheitshülle 28. Oberhalb der Leiterplatte 22 gibt es nochmals 0.5cm thermisch leitendes Silikon und dann einen Metallstab oder einen mit Silikon gefüllten Metallstab bis zur Oberseite des Gehäuses.

Wie Versuche gezeigt haben, spielt es für die Messung keine Rolle, ob der mindestens eine Wärmeflusssensor 20 und der mindestens eine Temperatursensor 21 auf der gleichen Seite der Leiterplatte 22 angeordnet sind oder gegenüberliegend. Die Messungen des Wärmeflusses mittels Wärmeflusssensor 20 und der Temperatur mittels Temperatursensor 21 werden genutzt, um die tatsächliche Körperkerntemperatur zu bestimmen.

In Figur 2a weist die Sensoreinheit 2 zwei Wärmeflusssensoren 20, 20' und einen Temperatursensor 21 auf einer hautzugewandten Seite der Leiterplatte 22 aufgelötet auf. Hier ist auf der Höhe der Längsrichtung eines Wärmeflusssensors 20 auf der wärmeflusssensorfreien Seite der Leiterplatte 22 eine thermische Isolation 23 angebracht. In der Variante gemäss Figur 2b ist auf der Höhe der Längsrichtung eines Wärmeflusssensors 20 auf der wärmeflusssensorfreien Seite der Leiterplatte 22 eine thermische Kapazität 24 angebracht. Damit wird ein Wärmefluss durch einen ersten Wärmeflusssensor 20 und eine thermische Isolation 23 bzw. thermische Kapazität 24 und ein Wärmefluss durch einen zweiten Wärmeflusssensor 20' gemessen. Beide Messwerte fliessen zusammen mit der Messung der Temperatur mittels Temperatursensor 21 in die Bestimmung der Körperkerntemperatur ein. Nach einer vorgängigen Kalibrierung der Wärmeflusssensormessung mit und ohne thermische Isolation 23 bzw. thermische Kapazität 24, kann so die Bestimmung der Körperkerntemperatur optimiert werden.

Die thermische Isolation 23 weist einen grossen thermischen Widerstand auf, wobei die Wärmeleitfähigkeit λ des Materials der thermischen Isolation 23 sehr klein ist. Wie in Figur 2a gezeigt, unterscheidet sich der Wärmefluss durch den ersten Wärmeflusssensor 20, aufgrund der thermischen Isolation 23 vom Wärmefluss durch den zweiten Wärmeflusssensor 20'. Als thermische Isolation 23 wird ein geschäumtes Kunststoffmaterial eingesetzt, wodurch der Wärmefluss durch ersten Wärmeflusssensor 20 kleiner ist, als durch den zweiten Wärmeflusssensor 20'. Entscheidend ist die Differenz der beiden Wärmeflüsse, woraus mittels Algorithmus die Bestimmung der Körperkerntemperatur verbessert bestimmbar ist. Durch diesen Aufbau ist die Sensoreinheit 2 robuster in Bezug auf Veränderungen der thermischen Ankopplung zwischen den Sensoren 20, 20', 21, 21' und der Haut H. Die Wärmestromdifferenz wird bei der Bestimmung berücksichtigt, weshalb die Eigenschaften der thermischen Isolation 23 bekannt sein müssen und experimentell bestimmt bzw. kalibriert werden. Die Wärmeleitfähigkeit λ des Materials der thermischen Isolation 23 sollte im Bereich von Luft (λ_{Luft} = 0.024 W/m/K), d.h. zwischen 0.01 W/m/K und 0.1 W/m/K liegen.

Als Materialien für die Sensoreinheitshülle 28 kommen Polymere, beispielsweise Polypropylene, PES, PE, PET, Polyethylen, Aceltal, Nylon, polybutylene terephthalate, Polysulfone, PPS, Polycarbonate, Teflon, Polyester, PMMA, PSU, PEEK, TPE, TPU, Parylene oder PTFE in Frage. Bevorzugt wurde Ethylen-Propylen-Dien-Kautschuk (EPDM) und Polypropylen gewählt. Auch die mindestens eine Leiterplatte 22 bzw. Material der Leiterplatte 22 kann Teil der Sensoreinheitshülle 28 sein. Als Material für die Leiterplatte 22 kann beispielsweise FR-4 oder FR4, ein Verbundwerkstoff aus Epoxidharz und Glasfasergewebe, verwendet werden. Wie dem Fachmann bekannt, kann die thermische Leitfähigkeit der Materialien durch spezielle Herstellung (zum Beispiel beigeben von Partikeln) verbessert oder so angepasst werden, so dass der Stoff eine ähnliche thermische Leitfähigkeit aufweist, wie die Sensoreinheit selber.

Die thermische Kapazität 24 hat entsprechend einen sehr geringen thermischen Widerstand und das Material eine sehr hohe Wärmeleitfähigkeit, ein sehr grosses λ. Die thermische Kapazität 24 sollte die Wärme maximal aufnehmen. Auch hier unterscheidet sich der Wärmefluss durch den ersten Wärmeflusssensor 20 vom Wärmefluss durch den zweiten Wärmeflusssensor 20'. Als Material wurde thermisch leitendes Silikon erfolgreich eingesetzt. Es können aber auch Metalle eingesetzt werden. Die Wärmeleitfähigkeit λ des Materials der thermischen Kapazität 24 sollte deutlich oberhalb der Wärmekapazität von Luft (λ_{Luft} = 0.024 W/m/K), d.h. grösser 0.1 W/m/K liegen.

Die Befestigung der thermischen Isolation 23 und der thermischen Kapazität 24 erfolgt mit einem geeigneten Kleber auf der Leiterplatte 22. Im Fall der Verwendung von Kupfer oder Messing kann dieses auf der Leiterplatte 22 aufgelötet sein.

Die Variante gemäss Figur 2c zeigt einen Wärmeflusssensor 20 und zwei Temperatursensoren 21, 21', wobei auf der Wärmeflusssensorseite der Leiterplatte 22 eine thermische Isolation 23 angebracht ist, welche auf gleicher Höhe in Längsrichtung der Leiterplatte 22, dem Temperatursensor 21' gegenüberliegt. Der Temperatursensor 21 ist direkt mit der Leiterplatte 22 verbunden, aber beabstandet von der thermischen Isolation 23. Damit sind zwei unterschiedliche Temperaturmessungen mittels zweier örtlich in Längsrichtung und Querrichtung beabstandet angeordneten Temperatursensoren 21, 21' möglich.

Die elektronischen Bauteile der Sensoreinheit 2, der mindestens eine Wärmeflusssensor 20, der mindestens eine Temperatursensor 21, Analog/Digital Wandler 25, Mikrokontroller 26 und Auslese-/Speichereinheit 27 können in einem integrierten Schaltkreis 29 (IC) verkapselt in einem Chipgehäuse zusammengefasst werden und mit der Leiterplatte 22 die Sensoreinheit 2 bilden. Die Verkapselung wird in der Praxis entweder durch ein beidseitiges Bekleben oder ein Eingiessen erreicht. Der integrierte Schaltkreis 29 bzw. das Chipgehäuse ist auf der Leiterplatte 22 aufgelötet. Durch die kompakte Bauweise des integrierten Schaltkreises 29, kann die Herstellung der Sensoreinheit 2 noch vereinfacht werden.

In Figur 2d ist eine Sensoreinheit 2 gezeigt, wobei zwei Wärmeflusssensoren 20, 20' auf derselben Seite der mindestens einen Leiterplatte 22 in Längsrichtung örtlich voneinander beabstandet angelötet sind. Auf der wärmeflusssensorfreien Seite der mindestens einen Leiterplatte 22 auf der Höhe des ersten Wärmeflusssensors 20 in Längsrichtung ist eine thermische Kapazität 24 angeordnet. Zusätzlich ist auf der wärmeflusssensorfreien Seite der mindestens einen Leiterplatte 22 auf der Höhe des zweiten Wärmeflusssensors 20' in Längsrichtung eine thermische Isolation 23 angeordnet. Eine Metallplatte 30 ist auf der wärmeflusssensorfreien Seite, die thermische Isolation 23 und die thermische Kapazität 24 berührend und parallel zur Leiterplatte 22 verlaufend angeordnet. Die Metallplatte 30 kann auch einen Block aus einem thermisch leitenden Material auf der Leiterplatte 22 oder die Sensoreinheitshülle 28 berührend bzw. teilweise überdeckend angeordnet sein.

Durch die Metallplatte 30, die bevorzugt aus Aluminium gefertigt und starr oder flexibel sein kann, wird ein thermisches Gleichgewicht mit derselben Aussentemperatur entlang der Metallplatte 30 erreicht und die Messgenauigkeit konnte erhöht werden.

Da bei der Integration der Sensoreinheit insbesondere in Uhren Platz gespart werden sollte, können thermische Isolation 23 und/oder thermische Kapazität 24 abgewandelt ausgestaltet werden. Dabei müssen thermische Isolation 23 bzw. Kapazität 24 nicht auf der Leiterplatte 22 angeordnet sein, sondern können auch in diese eingearbeitet sein. Hier sind beispielhaft eine thermische Isolation 23 innerhalb des Querschnitts der Leiterplatte 22 verlaufend dargestellt, wobei diese thermische Isolation eine Isolationslage beispielsweise ein Lufteinschluss sein kann. Die thermische Leitfähigkeit erhöht man durch das Einbringen von Thermovias, beispielsweise Cu vias, welche hier als thermische Kapazität 24 in Form von mit Kupfer gefüllten Sacklöchern bzw. Durchgangslöchern gestaltet sind. Ausserdem könnte eine thermische Kapazität 24 auch als verdecktes Thermovias, sogenannte "buried vias" ausgebildet sein, welche hier nicht dargestellt ist.

Wie in Figur 2f gezeigt ist die Leiterplatte 22 samt integriertem Schaltkreis 29 nahezu vollständig beidseitig von der Sensoreinheitshülle 28 umgeben. Der Anschluss der Verbindungskabel 3 an die Elektronik eines tragbaren Computersystems 0 ist ohne weiteres möglich.

Einsetzbare, vor allem auf der Leiterplatte 22 auflötbare Wärmeflusssensoren 20, 20', gehen aus der EP2938980 und der WO2016128247 des Anmelders hervor. Es sind miniaturisierte bzw. dünne monolithische Wärmeflusssensoren mit einer geringen thermischen Invasivität einzusetzen, die mechanisch ausreichend robust sind und sich auf die Oberfläche der Leiterplatte 22 auflöten lassen. Dazu wird ein sandwichartiger Aufbau aus mehreren Lagen unterschiedlicher Materialien aus Metallen und mindestens einer elektrisch isolierenden Matrixlage gewählt. Ein derartiger monolithischer Wärmeflusssensor 20, 20' bildet zu mindestens einer Seite einen geschlossenen Körper und eine Mehrzahl von Messübergängen zwischen verschiedenen Metalllagen. Temperatursensoren 21 sind schon seit langem bekannt und es können unterschiedlich gestaltete Temperatursensoren 21 eingesetzt werden. Als Temperatursensoren 21 kommen sämtliche bekannte Temperatursensoren 21 in Frage, die auf der Leiterplatte 22 oder einer weiteren Leiterplatte angelötet befestigbar sind. Unter anderem sind Infrarotsensoren zur Temperaturmessung einsetzbar, welche nicht von einer Sensoreinheitshülle 28 umgeben sein dürfen. In einem nicht erfindungsgemäßen Beispiel umfasst die Sensoreinheit 2 mehrere Leiterplatten 22, wobei der mindestens eine Wärmeflusssensor 20, 20' auf einer Leiterplatte 22 und ein Infrarotsensor zur Temperaturmessung auf einer weiteren Leiterplatte, örtlich von der ersten Leiterplatte 22 getrennt angeordnet ist. Mittels einer entsprechenden Verkabelung können Messwerte vom mindestens einen Wärmeflusssensor 20, 20' auf der ersten Leiterplatte 22 und Messwerte vom Infrarotsensor auf der zweiten Leiterplatte in der Auslese-/Speichereinheit 27 verarbeitet werden. Der Infrarotsensor ist zwar Teil der Sensoreinheit 2, muss aber frei von der Sensoreinheitshülle 28 bleiben. Am einfachsten wird das erreicht, wenn der Infrarotsensor auf der zweiten Leiterplatte angeordnet wird.

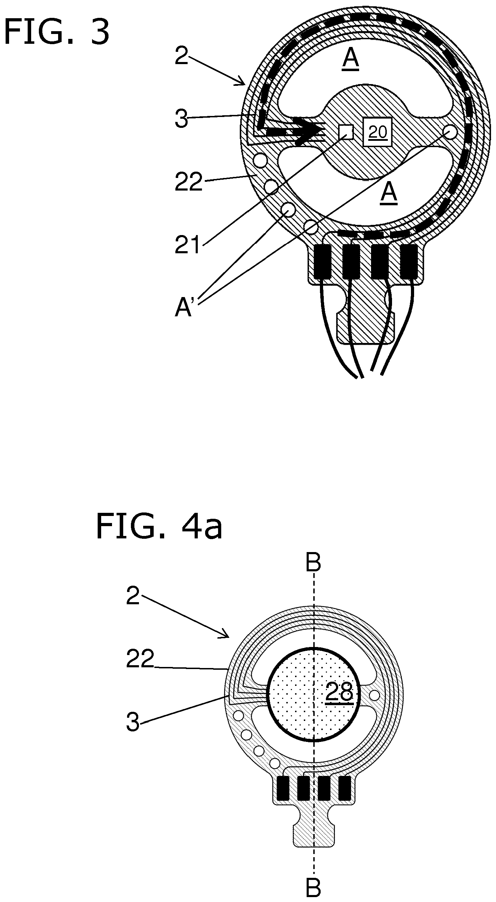

In Figur 3 ist eine Sensoreinheit 2 mit einer teilweise runden Leiterplatte 22 bzw. teilweise runder Querschnittsfläche mit Aussparungen A in einer Aufsicht auf die später zur Hautoberfläche weisenden Sensorseite gezeigt. Der Einfachheit halber ist hier eine Sensoreinheitshülle 28 weggelassen, sodass die von der Sensorseite wegragenden Wärmefluss- und Temperatursensoren 20, 21 sichtbar sind. Die Leiterbahnen auf der Leiterplatte 22 laufen entlang eines möglichst langen kreisförmigen Wegs, durch den gestrichelten Pfeil gekennzeichnet, entlang der Leiterplatte 22 bis zum Temperatursensor 21 und zum Wärmeflusssensor 20 im Zentrum der Leiterplatte 22. Damit werden thermische Wärmeverluste bzw. ein störender Wärmeeintrag aufgrund der Wärmeleitung der Verbindungskabel 3 nahezu unterbunden.

Vor allem durch die Anordnung der beiden nierenförmigen Aussparungen A, jeweils eine in Längsrichtung benachbart zum Wärmefluss- und Temperatursensor 20, 21, an einen Steg angrenzend, wodurch eine zentrische Leiterplattenscheibe gebildet wird, wird eine thermisch ungestörte Auflage des Temperatursensors 21 und des Wärmeflusssensors 20 erreicht. Durch die Aussparungen A' im Randbereich wird ein störender Wärmefluss zwischen Kontakten der Verbindungskabel 3 und den Sensoren 20, 21 auch noch zusätzlich verringert.

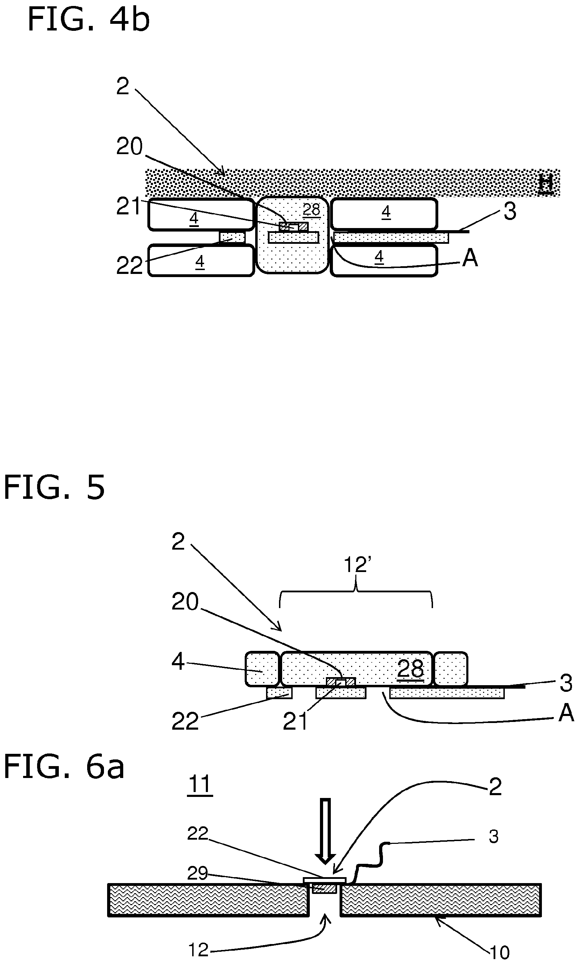

Eine mit einer Sensoreinheitshülle 28 versehene Sensoreinheit 2 ist in Figur 4a gezeigt. Die Sensoreinheitshülle 28 verdeckt die elektronischen Bauteile und die Leiterplatte 22 teilweise. Im Benutzungszustand gemäss Figur 4b, entlang der Schnittlinie B-B, ist die Sensoreinheit 2 mit Ihrer Sensoreinheitshülle 28 auf die Haut H eines Benutzers gelegt bzw. gepresst und damit kann die Messung von Wärmeflüssen und Temperaturen erfolgen und daraus die aktuelle Körperkerntemperatur mit bekannten Algorithmen bestimmt werden. Hier erstreckt sich die Sensoreinheitshülle 28 auch auf die der Haut abgewandten Seite der Leiterplatte 22 und die Sensoreinheitshülle 28 ist von einem Klebepad 4 umgeben.

Nach der Herstellung der Sensoreinheit 2 mit aufgebrachter Sensoreinheitshülle 28 kann die Leiterplatte 22 samt wegragender Sensoreinheitshülle 28 in eine Aussparung 12' im Klebepad 4, welches aus der Medizintechnik bekannt ist und beispielsweise zur Befestigung von Elektroden auf der Haut dient, befestigt werden. Die Verbindungskabel 3 können wahlweise vor oder nach der Befestigung der Sensoreinheit 2 im Klebepad 4 an eine Elektronik einer Medizintechnikvorrichtung angeschlossen werden. Das Klebepad 4 ist aus denselben Materialien hergestellt, wie die thermische Isolation 23 und weist eine hautverträgliche Klebeschicht, z.B. von der Firma 3M bekannt, auf.

Die Wärmeleitfähigkeit λ des Materials der thermischen des Kelebepad 4 sollte im Bereich von Luft (λ_{Luft} = 0.024 W/m/K), d.h. zwischen 0.01 W/m/K und 0.1 W/m/K liegen.

Um die Sensoreinheit 2 möglichst einfach in ein Gehäuse 1 eines tragbaren Computersystems 0 einzubringen, dienen im Folgenden beschriebene Verfahren.

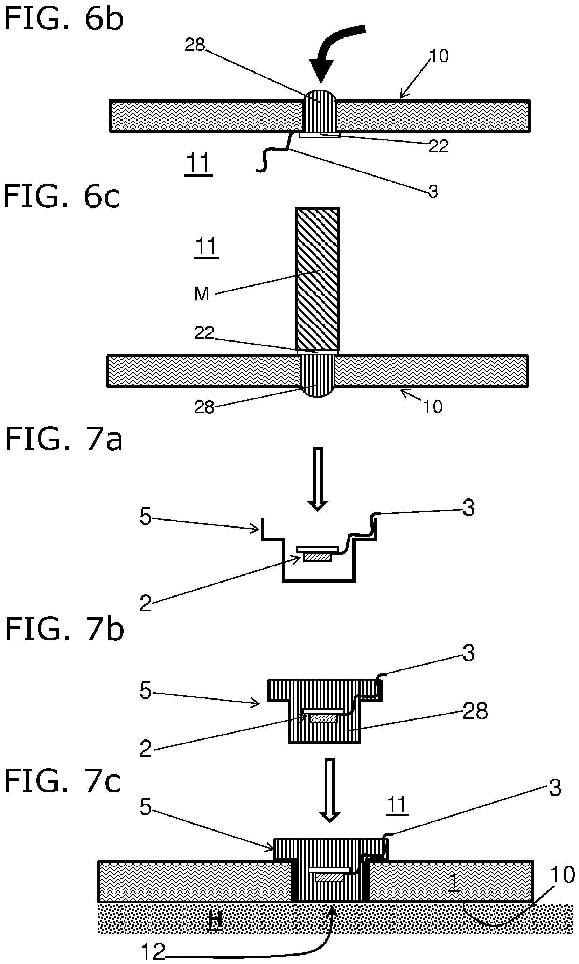

Bevorzugtes Herstellungs- bzw. Integrationsverfahren Anhand der Figuren 6 wird ein Integrationsverfahren der Sensoreinheit 2 in einem Gehäuse 1 eines tragbaren Computersystems 0 gezeigt. Dabei wird eine Sensoreinheit 2 derart zum Abdecken eines Durchgangsloches 12 im Gehäuse 1 verwendet, dass die Ränder der Leiterplatte 22 das Durchgangsloch 12 abschliessen. Die elektronischen Bauteile wie Wärmeflusssensor 20 und Temperatursensor 21 sind dabei auf der Höhe des Durchgangsloches 12 positioniert oder ragen in das Durchgangsloch 12 hinein. Die Verbindung der Leiterplatte 22 mit den Wänden des Gehäuses 1 kann mittels Klebstoff erfolgen. Durch die Abdeckung des Durchgangsloches 12 mit der Leiterplatte 22 kann in einem weiteren Schritt ein Ausfüllen des Durchgangsloches 12, beispielsweise mit einer Silikonmasse zur Bildung der Sensoreinheitshülle 28 erfolgen. Hier ist die Füllung in Figur 6b derart ausgeführt, dass ein Teil der Sensoreinheitshülle 28 aus dem Durchgangsloch 12 herausragt in Richtung Hautkontaktseite 10. Die Positionierung der Leiterplatte 22 wird wie mit dem Pfeil gekennzeichnet, durchgeführt. Die Sensoreinheit 2 ist wie oben beschrieben ausgeführt, wird aber erst nach Fixierung der Leiterplatte 22 mit der Sensoreinheitshülle 28 versehen. Das Auffüllen des Durchgangslochs 12 findet aus Richtung des Innenraums 11 des Gehäuses 1 statt.

Die Verbindungskabel 3 von der Leiterplatte 22 zur nicht dargestellten Elektronik können wahlweise vor dem Eingiessen oder Befüllen verbunden werden oder erst nach dem Befüllen der Aussparung 12. Um eine beschleunige Wärmeleitung und eine Equilibrierung zwischen Umgebung und Körper zu erhalten, ist auf der dem Körper abgewandten Seite der Leiterplatte 22, in Richtung des Innenraums 11 weisend und in Höhe des Wärmeflusssensors 20 ein Metallstab M angebracht, der den Innenraum 11 des Gehäuses 1 quert und mit der Aussenseite des Gehäuses 1 in Kontakt steht Je nach Ausführungsform kann dieser Metallstab M an seinen Seitenwänden auch thermisch isoliert sein.

Es ist auch ein weiteres Herstellungsverfahren möglich, welches in Figuren 7a bis 7c beschrieben ist. Nach der Herstellung der Sensoreinheit 2 wird dieses in einer Gussform 5 platziert, wobei die Verbindungskabel 3 aus der Gussform 5 hinausragen. Die Wände der Gussform 5 können wahlweise aus Metall oder Kunststoffmaterial geformt sein.

Im nächsten Schritt wird die Gussform 5 mit einem Material gefüllt, welches später die Sensoreinheitshülle 28 bildet. Die Sensoreinheit 2 wird dabei entsprechend von allen Seiten von dem Material umschlossen, sodass die Leiterplatte 22 und alle Bauteile von der Sensoreinheitshülle 28 umschlossen sind. Nach dem Aushärten kann die umschlossene Sensoreinheit 2 in eine Aussparung 12, 13 im Gehäuse 1 eines tragbaren Computersystems 0 platziert und darin lösbar gehalten eingepresst werden. Das thermisch ausreichend leitfähige Material der Sensoreinheitshülle 28 schmiegt sich an die Wände der Aussparung 12, 13 an, wodurch ein ungewolltes einfaches Herausrutschen unterbunden ist. Die in der Sensoreinheitshülle 28 eingebettete Sensoreinheit 2 bildet einen Pfropfen, der in der Aussparung 12, 13 verbleibt.

Um das Halten zu verbessern, kann ein geeigneter Kleber verwendet werden, der vor dem Einpressen der mit der Sensoreinheitshülle 28 umhüllten Sensoreinheit 2 an den Wänden der Aussparung 12, 13 verteilt wird. Entsprechend ist auch eine unlösbare Verbindung erreichbar.

Die hier verwendet Gussform 5 kann wahlweise als verlorene Gussform verwendet, mit der Sensoreinheit 2 verbunden bleiben oder entfernt werden, bevor die Sensoreinheit 2 in der Aussparung 12 im Gehäuse 1 befestigt wird.

In der Praxis werden bei auf die Haut H aufgelegter Sensoreinheit 2, Messwerte von dem mindestens einen Wärmeflusssensor 20, 20' und dem mindestens einen Temperatursensor 21, 21' aufgenommen, woraus mittels unterschiedlicher Algorithmen die Körperkerntemperatur ermittelt werden kann. Zur möglichst genauen Bestimmungen sind Kalibriermessungen nötig und die verwendeten Materialienarten und - dicken müssen bekannt sein. Es sind mehrere mögliche Algorithmen bekannt, die zu brauchbaren Ergebnissen bei der Bestimmung der Körperkerntemperatur führen.

Bevorzugt ist die Sensoreinheit 2 derart in die Sensoreinheitshülle 28 eingebracht, dass die Sensoreinheitshülle 28 teilweise aus dem Gehäuse 1 von den Wänden des Gehäuses 1 wegragend angeordnet ist. Entsprechend ist dann die thermische Ankopplung der Sensoreinheit 2 bzw. der Sensoren 20, 21 optimiert.

Die Sensoreinheitshülle 28 könnte eine Hülse oder ein Zylinder beispielsweise aus PVC sein, welche mit dem thermisch leitfähigen Material gefüllt ist. Dieser Zylinder könnte auch aus Isolationsmaterial (Schaumstoff) bestehen, um den thermischen Pfad besser zu definieren.

In einer nicht erfindungsgemäßen Variante kann die Sensoreinheit 2 eben mehrere voneinander getrennte Leiterplatten 22 aufweisen, auf welchen Temperatursensoren 21, 21' und Wärmeflussensoren 20, 20' und/oder andere elektronische Bauteile voneinander getrennt angeordnet sein können. Dann sollten alle Leiterplatten 22 entsprechend mindestens teilweise von mindestens einer Sensoreinheitshülle 28 umgeben sein. Im Fall der Benutzung eines Infrarotsensors als Temperatursensor 21, 21' darf dieser nicht von der Sensoreinheitshülle 28 umgeben sein.

### Bezugszeichenliste

0 tragbares Computersystem
1 Gehäuse
   10 Hautkontaktseite
   11 Innenraum
   12 Aussparung/Durchgangsloch
   13 Aussparung/Sackloch
2 Sensoreinheit
   20, 20' Wärmeflusssensor (mindestens 1)
   21, 21' Temperatursensor (mindestens 1)
   22 Leiterplatte /(printed circuit board, PCB)
   23 thermische Isolation
   24 thermische Kapazität
   25 Analog/Digital Wandler
   26 Mikrokontroller
   27 Auslese-/Speichereinheit
   28 Sensoreinheitshülle
   29 integrierter Schaltkreis, verpackt
   30 Metallplatte
   A Aussparung
3 Verbindungskabel (zur Elektronik)
4 Klebepad (zum Aufbringen auf die Haut)
5 Gussform

## Patentansprüche

1. Sensoreinheit (2) zur Bestimmung der Körperkerntemperatur durch ausserhalb des Körpers auf einer Oberfläche ermittelbare Messwerte, umfassend einen Analog/Digital Wandler (25), einen Mikrokontroller (26), eine Auslese-/Speichereinheit (27), sowie mindestens einen monolithischen Wärmeflusssensor (20, 20') und mindestens einen Temperatursensor (21, 21'),
wobei die Sensoreinheit (2) mindestens eine Leiterplatte (22) umfasst,
wobei der mindestens eine monolithische Wärmeflusssensor (20, 20') in Form eines sandwichartigen Aufbaus aus mehreren Lagen unterschiedlicher Materialien aus Metallen und
mindestens einer elektrisch isolierenden Matrixlage bestehend, einen zu mindestens einer Seite geschlossenen Körper und eine Mehrzahl von Messübergängen zwischen verschiedenen Metalllage bildet,
der mindestens eine monolithische Wärmeflusssensor (20) und der mindestens eine Temperatursensor (21) auf Lötflächen auf der Leiterplatte (22) platziert und mittels Lot auf derselben Seite der Leiterplatte (22) voneinander örtlich beabstandet befestigt sind, und wobei
der mindestens eine monolithische Wärmeflusssensor (20, 20') und der mindestens eine Temperatursensor (21, 21') mit dem Analog/Digital Wandler (25), dem Mikrokontroller (26) und der Auslese-/Speichereinheit (27) via Kabel oder Leiterbahnen verbunden sind, wobei der mindestens eine monolithischen Wärmeflusssensor (20, 20'), der mindestens eine Temperatursensor (21, 21'), der Analog/Digital Wandler (25), der Mikrokontroller (26) und die Auslese-/Speichereinheit (27) mittels Verbindungskabeln (3) oder Leiterbahnen an eine Elektronik eines tragbaren Computersystems (0) anschliessbar sind und die mindestens eine Leiterplatte (22) mit darauf angeordneten monolithischen Wärmeflusssensor (20, 20'), Temperatursensor (21, 21') und Analog/Digital Wandler (25) mindestens teilweise von einer Sensoreinheitshülle (28) umgeben sind.

2. Sensoreinheit (2) nach Anspruch 1 wobei die Sensoreinheit (2) und/oder die mindestens eine Leiterplatte (22) vollständig von der Sensoreinheitshülle (28) umhüllt sind.

3. Sensoreinheit (2) nach einem der vorhergehenden Ansprüche, wobei neben dem mindestens einen monolithischen Wärmeflusssensor (20) ein zweiter monolithischer Wärmeflusssensor (20') auf derselben Seite der mindestens einen Leiterplatte (22) in einer Längsrichtung örtlich voneinander beabstandet angelötet ist und auf der wärmeflusssensorfreien Seite der Leiterplatte (22) in einer Längsrichtung auf einer Höhe des ersten monolithischen Wärmeflusssensors (20) eine thermische Isolation (23) angeordnet ist, sodass ein erster Wärmefluss durch die thermische Isolation (23) und den ersten monolithischen Wärmeflusssensor (20) und ein zweiter Wärmefluss durch den zweiten monolithischen Wärmeflusssensor (20') messbar ist.

4. Sensoreinheit (2) nach einem der Ansprüche 1 oder 2, wobei zwei monolithische Wärmeflusssensoren (20, 20') auf derselben Seite der mindestens einen Leiterplatte (22) in einer Längsrichtung der Leiterplatte (22) örtlich voneinander beabstandet angelötet sind und auf einer wärmeflusssensorfreien Seite der mindestens einen Leiterplatte (22) auf der Höhe des ersten monolithischen Wärmeflusssensors (20) in Längsrichtung der Leiterplatte (22) eine thermische Kapazität (24) angeordnet ist, sodass ein erster Wärmefluss durch die thermische Kapazität (24) und den ersten monolithischen Wärmeflusssensor (20) und ein zweiter Wärmefluss durch den zweiten monolithischen Wärmeflusssensor (20') messbar ist.

5. Sensoreinheit (2) nach Anspruch 1, wobei auf einer wärmeflusssensorfreien Seite der mindestens einen Leiterplatte (22) eine Metallplatte (30) und/oder eine thermische Isolation (23) und/oder eine thermische Kapazität (24) und/oder ein Block aus einem thermisch leitenden Material einen Teil der Sensoreinheitshülle (28) berührend verlaufend angeordnet ist.

6. Sensoreinheit (2) nach einem der vorhergehenden Ansprüche, wobei auf einer Seite der mindestens einen Leiterplatte (22) der mindestens eine monolithische Wärmeflusssensor (20), der mindestens eine Temperatursensor (21') als erster Temperatursensor (21') und eine thermische Isolation (23) örtlich in einer Längsrichtung der Leiterplatte (22) voneinander beabstandet sind und auf einer gegenüberliegenden wärmeflusssensorfreien Seite der mindestens einen Leiterplatte (22) ein zweiter Temperatursensor (21) entlang der Längsrichtung der Leiterplatte (22) in Höhe der thermischen Isolation (23) angeordnet ist, sodass eine erste Temperatur am Ort des ersten Temperatursensors (21') und eine zweite Temperatur am Ort des zweiten Temperatursensors (21) nach Durchgang des Wärmeflusses durch die thermische Isolation (23) messbar sind.

7. Sensoreinheit (2) nach Anspruch 1, wobei eine thermische Isolation (23) und/oder eine thermische Kapazität (24) innerhalb der Leiterplatte (22) verlaufend angeordnet ist und durch einen Isolationslageneinschluss (23), bevorzugt einen Lufteinschluss und/oder durch Thermovias (24) gebildet ist.

8. Tragbares Computersystem (0) mit einer Sensoreinheit (2) nach einem der vorhergehenden Ansprüche.

9. Integrationsverfahren einer Sensoreinheit (2) nach einem der Ansprüche 1 bis 7, umfassend mindestens einen monolithischen Wärmeflusssensor (20, 20'), mindestens einen Temperatursensor (21, 21') und einen Analog/Digital Wandler (25) auf einer Leiterplatte (22) in ein Gehäuse (1) eines tragbaren Computersystems (0), umfassend **die Schritte:**
- Bereitstellen eines Gehäuses (1) mit einer Aussparung (12) in Form eines Durchgangsloches (12),
- Bereitstellen der Sensoreinheit (2), umfassend eine Leiterplatte (22) von welcher der mindestens eine monolithische Wärmeflusssensor (20, 20') wegragt, wobei der mindestens eine monolithische Wärmeflusssensor (20, 20') mit dem Analog/Digital Wandler (25), einem Mikrokontroller (26) und einer Auslese-/Speichereinheit (27) verbunden sind,
- Platzieren der Leiterplatte (22) über dem Durchgangsloch (12) im Gehäuse (1) und Befestigung der Leiterplatte (22) an den Wänden des Gehäuses (1),
- Auffüllen des einseitig verschlossenen Durchgangsloches (12) mit einem fliessfähigen Material, welches eine Sensoreinheitshülle (28) bildet und anschliessende
- Verbindung der Leiterplatte (22) mittels Verbindungskabeln (3) an einer Elektronik des tragbaren Computersystems (0).

10. Herstellung einer Sensoreinheit (2) nach einem der Ansprüche 1 bis 7, umfassend mindestens einen monolithischen Wärmeflusssensor (20, 20'), mindestens einen Temperatursensor (21, 21') und einen Analog/Digital Wandler (25), auf einer Leiterplatte (22) und Integration der Sensoreinheit (2) in ein Gehäuse (1) eines tragbaren Computersystems (0) oder in ein Klebepad (4) zum Anbringen auf der Haut (H), umfassend **die Schritte:**
- Bereitstellen eines Gehäuses (1) mit einer Aussparung (12, 13) respektive eines Klebepads (4) mit einer Aussparung (12'),
- Bereitstellen der Sensoreinheit (2)
- Einfügen der Sensoreinheit (2) in eine Gussform (5), wobei Verbindungskabel (3) aus der Gussform (5) herausragen,
- Giessen eines mechanisch flexibel aushärtenden Materials, bevorzugt eines thermisch leitenden Silikons in die Gussform (5) zur Formung einer Sensoreinheitshülle (28),
- Aushärten der Sensoreinheitshülle (28),
- Einfügen der von der Sensoreinheitshülle (28) umschlossenen Sensoreinheit (2) in die Aussparung (12, 13) im Gehäuse (1) des tragbaren Computersystems (0) respektive der Aussparung (12') im Klebepad (4) und
- anschliessende Verbindung der Verbindungskabel (3) mit einer Elektronik des tragbaren Computersystems (0).

## Claims

1. A sensor unit (2) for determining core body temperature by measurable values detected on a surface outside the body, comprising an analog/digital converter (25), a microcontroller (26), a reading/storage unit (27) and at least one monolithic heat flux sensor (20, 20') and at least one temperature sensor (21, 21'),
wherein the sensor unit (2) comprises at least one printed circuit board (22), wherein the at least one monolithic heat flux sensor (20, 20') consists of a sandwich-like structure made of multiple layers of different materials from metals and at least one electrically insulating matrix layer, forming a body that is closed on at least one side and a plurality of measurement junctions between different metal layers,
the at least one monolithic heat flux sensor (20) and the at least one temperature sensor (21) are placed on soldering pads on the printed circuit board (22) and are fixed spatially separate from one another on the same side of the printed circuit board (22) by means of solder, and wherein
the at least one monolithic heat flux sensor (20, 20') and the at least one temperature sensor (21, 21') are connected via cables or circuit traces to the analog/digital converter (25), the microcontroller (26), and the reading/storage unit (27), wherein the at least one monolithic heat flux sensor (20, 20'), the at least one temperature sensor (21, 21'), the analog/digital converter (25), the microcontroller (26) and the reading/storage unit (27) can be connected to the electronics of a portable computer system (0) by means of connecting cables (3) or circuit traces and the at least one printed circuit board (22) with the monolithic heat flux sensors (20, 20'), temperature sensors (21, 21') and analog/digital converter (25) arranged thereon are at least partially enclosed by a sensor unit casing (28).

2. The sensor unit (2) according to claim 1, wherein the sensor unit (2) and/or the at least one printed circuit board (22) are completely enclosed by the sensor unit casing (28).

3. The sensor unit (2) according to any one of the preceding claims, wherein in addition to the at least one monolithic heat flux sensor (20), a second monolithic heat flux sensor (20') is soldered on the same side of the at least one printed circuit board (22), spaced apart from one another in a longitudinal direction, and on the heat-flux-sensor-free side of the printed circuit board (22), thermal insulation (23) is arranged in the longitudinal direction at the height of the first monolithic heat flux sensor (20), so that a first heat flux through the thermal insulation (23) and the first monolithic heat flux sensor (20) and a second heat flux through the second monolithic heat flux sensor (20') are measurable.

4. The sensor unit (2) according to any one of claims 1 or 2, wherein two monolithic heat flux sensors (20, 20') are soldered on the same side of the at least one printed circuit board (22), spaced apart from one another in a longitudinal direction of the printed circuit board (22), and on the heat-flux-sensor-free side of the at least one printed circuit board (22), a thermal capacity (24) is arranged in the longitudinal direction of the printed circuit board (22) at the height of the first monolithic heat flux sensor (20), so that a first heat flux through the thermal capacity (24) and the first monolithic heat flux sensor (20) and a second heat flux through the second monolithic heat flux sensor (20') are measurable.

5. The sensor unit (2) according to claim 1, wherein on a heat-flux-sensor-free side of the at least one printed circuit board (22), a metal plate (30) and/or thermal insulation (23) and/or a thermal capacity (24) and/or a block made of a thermally conductive material is arranged in contact with part of the sensor unit casing (28).

6. The sensor unit (2) according to any one of the preceding claims, wherein on one side of the at least one printed circuit board (22), the at least one monolithic heat flux sensor (20), the at least one temperature sensor (21') as the first temperature sensor (21') and thermal insulation (23) are spaced apart from one another in a longitudinal direction of the printed circuit board (22), and on an opposite heat-flux-sensor-free side of the at least one printed circuit board (22), a second temperature sensor (21) is arranged along the longitudinal direction of the printed circuit board (22) at the height of the thermal insulation (23), so that a first temperature at the location of the first temperature sensor (21') and a second temperature at the location of the second temperature sensor (21) can be measured after the heat flux has passed through the thermal insulation (23).

7. The sensor unit (2) according to claim 1, wherein thermal insulation (23) and/or a thermal capacity (24) is/are arranged within the printed circuit board (22) extending through it and is formed by an insulation layer inclusion (23), preferably an air inclusion and/or by thermovias (24).

8. A portable computer system (0) with a sensor unit (2) according to any one of the preceding claims.

9. An integration method of a sensor unit (2) according to any one of claims 1 to 7, comprising at least one monolithic heat flux sensor (20, 20'), at least one temperature sensor (21, 21') and an analog/digital converter (25) on a printed circuit board (22) into a housing (1) of a portable computer system (0), comprising the steps:
- providing a housing (1) with a recess (12) in the form of a through-hole (12),
- providing the sensor unit (2), comprising a printed circuit board (22) from which the at least one monolithic heat flux sensor (20, 20') protrudes, wherein the at least one monolithic heat flux sensor (20, 20') is connected to the analog/digital converter (25), a microcontroller (26) and a reading/storage unit (27),
- placing the printed circuit board (22) over the through-hole (12) in the housing (1) and attaching the printed circuit board (22) to the walls of the housing (1),
- filling the one-sidedly closed through-hole (12) with a flowable material which forms a sensor unit casing (28) and subsequently
- connecting the printed circuit board (22) by means of connection cables (3) to the electronics of the portable computer system (0).

10. Production of a sensor unit (2) according to any one of claims 1 to 7, comprising at least one monolithic heat flux sensor (20, 20'), at least one temperature sensor (21, 21') and an analog/digital converter (25) on a printed circuit board (22) and integrating the sensor unit (2) into a housing (1) of a portable computer system (0) or into an adhesive pad (4) for attachment to the skin (H), comprising the steps:
- providing a housing (1) with a recess (12, 13) or an adhesive pad (4) with a recess (12'),
- providing the sensor unit (2),
- inserting the sensor unit (2) into a mould (5), wherein connection cables (3) protrude from the mould (5),
- casting a mechanically flexible hardening material, preferably a thermally conductive silicone, into the mould (5) to form a sensor unit casing (28),
- hardening the sensor unit casing (28),
- inserting the sensor unit (2) enclosed by the sensor unit casing (28) into the recess (12, 13) in the housing (1) of the portable computer system (0) or the recess (12') in the adhesive pad (4), and
- subsequently connecting the connection cables (3) to the electronics of the portable computer system (0).

## Revendications

1. Unité de capteur (2) pour déterminer la température corporelle centrale par des valeurs de mesure détectables sur une surface à l'extérieur du corps, comprenant un convertisseur analogique/numérique (25), un microcontrôleur (26), une unité de lecture/mémorisation (27), et au moins un capteur de flux de chaleur monolithique (20, 20') et au moins un capteur de température (21, 21'),
dans laquelle l'unité de capteur (2) comprend au moins une plaquette de circuit imprimé (22), dans laquelle le au moins un capteur de flux thermique monolithique (20, 20') forme un corps fermé sur au moins un côté et une pluralité de transitions de mesure entre différentes couche métallique, sous la forme d'une structure de type sandwich composée de plusieurs couches de différents matériaux constituées de métaux et d'au moins une couche matricielle électriquement isolante, le ou les capteurs de flux de chaleur monolithiques (20) et le ou les capteurs de température (21) sont placés sur des surfaces de soudure sur la plaquette de circuit imprimé (22) et sont fixés au moyen de soudures sur le même côté de la plaquette de circuit imprimé (22) à une distance spatiale l'un de l'autre, et dans laquelle au moins un capteur de flux thermique monolithique (20, 20') et au moins un capteur de température (21, 21) sont connectés avec le convertisseur analogique/numérique (25), le microcontrôleur (26) et une unité de lecture/mémorisation (27) via des câbles ou des pistes conductrices, dans laquelle le au moins un capteur de flux de chaleur monolithique (20, 20'), qui comporte au moins un capteur de température (21, 21'), le convertisseur analogique/numérique (25), le microcontrôleur (26) et l'unité de lecture/mémorisation (27) peuvent être connectés à l'électronique d'un système informatique portable (0) au moyen de câbles de connexion (3) ou de pistes conductrices et la moins une plaquette de circuit imprimé (22) sur laquelle est disposé un capteur de flux thermique monolithique (20, 20), un capteur de température (21, 21) et un convertisseur analogique/numérique (25) sont au moins partiellement entourés par une coque d'unité de capteur (28).

2. Unité de capteur (2) selon la revendication 1, dans laquelle l'unité de capteur (2) et/ou la ou les cartes de circuits imprimés (22) sont entièrement recouvertes par la coque d'unité de capteur (28).

3. Unité de capteur (2) selon une des revendications précédentes, dans laquelle, en plus du au moins un capteur de flux de chaleur monolithique (20) un deuxième capteur de flux thermique monolithique (20) est soudé sur le même côté d'au moins une plaquette de circuit imprimé (22) dans la direction longitudinale à distance l'un de l'autre et sur le côté exempt de capteur de flux thermique de la plaquette de circuit imprimé (22) dans une direction longitudinale à une hauteur du premier capteur de flux thermique monolithique (20), une isolation thermique (23) est disposée de telle sorte qu'un premier flux de chaleur traversant l'isolation thermique (23) et le premier capteur de flux thermique monolithique (20) et un deuxième flux de chaleur traversant le deuxième capteur de flux thermique monolithique (20') puisse être mesuré.

4. Unité de capteur (2) selon une des revendications 1 ou 2, dans laquelle deux capteurs de flux thermique monolithiques (20, 20') sont soudés sur le même côté d'au moins une plaquette de circuit imprimé (22) dans une direction longitudinale de la plaquette de circuit imprimé (22) à une distance spatiale l'un de l'autre et une capacité thermique (24) est disposée sur un côté exempt de capteur de flux thermique de la au moins une plaquette de circuit imprimé (22) à la hauteur du premier capteur de flux thermique monolithique (20) dans la direction longitudinale de la plaquette de circuit imprimé (22), de sorte qu'un premier flux de chaleur traversant la capacité thermique (24) et le premier capteur de flux de chaleur monolithique (20) et un deuxième flux de chaleur traversant le deuxième capteur de flux de chaleur monolithique (20') puissent être mesurés.

5. Unité de capteur (2) selon la revendication 1, dans laquelle, sur un côté exempt de capteur de flux thermique de la au moins une plaquette de circuit imprimé (22) une plaque métallique (30) et/ou une isolation thermique (23) et/ou une capacité thermique (24) et/ou un bloc constitué d'un matériau thermoconducteur est disposé au contact d'une partie de la coque d'unité de capteur (28).

6. Unité de capteur (2) selon une des revendications précédentes, dans laquelle sur un côté de la au moins une plaquette de circuit imprimé (22) se trouve au moins un capteur de flux de chaleur monolithique (20), le au moins un capteur de température (21') en tant que premier capteur de température (21) et l'isolation thermique (23) sont espacés l'un de l'autre localement dans une direction longitudinale de la plaquette de circuit imprimé (22) et sur un côté opposé exempt de capteur de flux de chaleur de la ou des plaquettes de circuit imprimé (22) un deuxième capteur de température (21) est disposé le long de la direction longitudinale de la plaquette de circuit imprimé (22) à la hauteur de l'isolation thermique (23) de telle sorte qu'une première température puisse être mesurée à l'emplacement du premier capteur de température (21) et une deuxième température puisse être mesurée à l'emplacement du deuxième capteur de température (21) après le passage du flux de chaleur à travers l'isolation thermique (23).

7. Unité de capteur (2) selon la revendication 1, dans laquelle une isolation thermique (23) et/ou une capacité thermique (24) est disposée à l'intérieur de la plaquette de circuit imprimé (22) et est formée via une inclusion de couche d'isolation (23), de préférence une inclusion d'air et/ou par des vias thermiques (24).

8. Système informatique portable (0) doté d'une unité de capteur (2) selon une des revendications précédentes.

9. Procédé d'intégration (2) selon une des revendications 1 à 7, comprenant au moins un capteur de flux de chaleur monolithique (20, 20'), au moins un capteur de température (21, 21') et un convertisseur analogique/numérique (25) sur une plaquette de circuit imprimé (22) dans un boîtier (1) d'un système informatique portable (0), comprenant les étapes consistant à :
- munir un boîtier (1) d'un évidement (12) en forme de trou traversant (12),
- fournir l'unité de capteur (2), comprenant une plaquette de circuit imprimé (22) à partir de laquelle dépasse en saillie le au moins un capteur de flux thermique monolithique (20, 20'), dans lequel le au moins un capteur de flux thermique monolithique (20, 20') est connecté au convertisseur analogique/numérique (25), à un microcontrôleur (26) et à une unité de lecture/mémorisation (27),
- placer la plaquette de circuit imprimé (22) sur le trou traversant (12) dans le boîtier (1) et fixer le circuit imprimé (22) aux parois du boîtier (1),
- remplir le trou traversant (12), qui est fermé d'un côté, avec un matériau fluide, qui forme une coque d'unité de capteur (28) et ensuite
- connecter la plaquette de circuit imprimé (22) à l'aide de câbles de liaison (3) à l'électronique du système informatique portable (0).

10. Fabrication d'une unité de capteur (2) selon une des revendications 1 à 7, Z comprenant au moins un capteur de flux de chaleur monolithique (20, 20'), au moins un capteur de température (21, 21') et un convertisseur analogique/numérique (25), sur une plaquette de circuit imprimé (22) et intégration de l'unité de capteur (2) dans un boîtier (1) d'un système informatique portable (0) ou dans une pastille adhésive (4) de fixation sur la peau (H), comprenant de :
- munir un boîtier (1) d'un évidement (12, 13) ou une pastille adhésive (4) d'un évidement (12),
- fournir l'unité de capteur (2),
- insérer l'unité de capteur (2) dans un moule (5), dans lequel des câbles de liaison (3) dépassent du moule (5),
- couler un matériau durcissant mécaniquement flexible, de préférence un silicone thermoconducteur, dans le moule (5) pour former une coque d'unité de capteur (28),
- durcir la coque d'unité de capteur (28),
- insérer l'unité de capteur (2) entourée par la coque de l'unité de capteur (28) dans l'évidement (12, 13) du boîtier (1) du système informatique portable (0) ou dans l'évidement (12') dans la pastille adhésive (4) et
- brancher ultérieurement le câble de liaison (3) avec une électronique du système informatique portable (0).
